(19) Europäisches Patentamt
European Patent Office
Office européen des brevets

(11) **EP 0 669 140 B1**

(12) **EUROPÄISCHE PATENTSCHRIFT**

(45) Veröffentlichungstag und Bekanntmachung des Hinweises auf die Patenterteilung:
**19.05.1999 Patentblatt 1999/20**

(51) Int. Cl.$^6$: **A61M 13/00**

(21) Anmeldenummer: **95100038.9**

(22) Anmeldetag: **03.01.1995**

(54) **Insufflationsgerät**

Insufflator

Insufflateur

(84) Benannte Vertragsstaaten:
**CH DE FR GB LI**

(30) Priorität: **28.01.1994 DE 4402467**

(43) Veröffentlichungstag der Anmeldung:
**30.08.1995 Patentblatt 1995/35**

(73) Patentinhaber: **Richard Wolf GmbH**
**75438 Knittlingen (DE)**

(72) Erfinder:
• **Schneider, Herbert, Dipl.-Ing.**
**D-75417 Mühlacker (DE)**

• **Rentschler, Gunter**
**D-76703 Kraichtail-Münzesheim (DE)**

(74) Vertreter:
**Wilcken, Thomas, Dipl.-Ing.**
**Patentanwälte**
**Wilcken & Vollmann**
**Musterbahn 1**
**23552 Lübeck (DE)**

(56) Entgegenhaltungen:
**EP-A- 0 384 155**     **EP-A- 0 517 190**
**DE-C- 3 611 018**     **FR-A- 2 326 734**
**GB-A- 2 271 427**     **US-A- 5 549 546**

**Beschreibung**

Die Erfindung betrifft ein Insufflationsgerät, insbesondere zum Insufflieren von Gas in das Cavum Uteri, mit einem Gas liefernden Reservoir und mit einer Regelvorrichtung, mit der Sensoren und ein Stellglied verbunden sind, um den Druck und den Flow des in eine Körperhöhle eingeleiteten Gases zu messen und auf vorgebbare Werte einzustellen und um die Gaszufuhr nach Erreichen eines als Maximalwert vorgesehenen Gasdrucks in der Körperhöhle zu beenden.

Ein solches Insufflationsgerät ist beispielsweise aus der DE-C-3 611 018 und der DE-C-39 22 746 bekannt. Bei diesem Insufflationsgerät ist einem Gassteuerkreis ein Absaugkreis zugeordnet, der eine Pumpe umfaßt, deren Saugseite mit einem Durchflußmesserausgang verbunden ist und deren Druckseite an einem Endoskop angeschlossen ist. Ein Druckmeßwandler registriert den Druck an der Saugseite der Pumpe, wobei die Meßdaten mit denen des Durchflußmessers in einer Auswerteelektronik so verwertet werden, daß beim Auftreten einer Störung im Absaugkreis die Pumpe abgeschaltet wird.

Bei der Ausführung einer Hysteroskopie wird der Uterus durch ein Insufflationsgerät über eine Sonde oder einen Endoskopkanal mit Gas gefüllt. Dabei entfaltet sich das Cavum Uteri, und es stellt sich ein bestimmter intrakavitärer Druck ein. Dieser liegt bei normalen anatomischen Verhältnissen insbesondere zwischen 0,5 bis 1 x $10^4$ Pa, und der Gasflow (Massenfluß) liegt zwischen 30 bis 40 ml/min. Das Insufflationsgerät führt automatisch nur noch die Gasmenge zu, die gegebenenfalls gleichzeitig durch die Tuben in die Bauchhöhle entweicht. Wenn das Hysteroskop bei der Untersuchung hin und her geschoben wird, kann der intrakavitäre Druck wechseln. Jedoch tritt schon nach kurzer Zeit ein Druckgleichgewicht wieder ein. Die zufließende Gasmenge wird dabei normalerweise umso kleiner, je höher der Druck in der Körperhöhle steigt. Besteht Druckausgleich zwischen Insufflationsgerät und Cavum Uteri, fließt auch kein Gas mehr. Bei verschlossenen Tuben steigt dann der intrauterine Druck auf die Höhe des vorgegebenen Insufflationsdrucks an, während der Flow auf Null zurückgeht.

Zur Schonung der Patientin darf bei der Untersuchung ein gegebener maximaler Gasdruck nicht überschritten werden. Dies wird im Simulationsbetrieb überprüft, wobei der Gasflow nicht mehr als 70 oder 100 ml/min und der Insufflationsdruck nicht mehr als 1,99 bis 2,66 x $10^4$ Pa betragen dürfen.

Im Normalbetrieb wird die Bedienperson somit nach anfänglicher Überprüfung des Insufflationsgeräts, d.h. mittels des oben erwähnten simulierten Betriebs, und dann in echtem Betrieb an den vorgesehenen Anzeigen des Geräts die Einhaltung vorgegebener Druck- und Gasflowwerte überwachen und eine entsprechende Einstellung an Ventilen des Gerätes vornehmen.

Um die Bedienperson, z.B. den untersuchenden Arzt, von solchen seine Aufmerksamkeit ablenkenden Ablesungen der Instrumente zu befreien, ist ein automatischer Betrieb eines solches Insufflationsgerätes wünschenswert, so daß die Einhaltung der vorgegebenen Höchstwerte von Druck und Gasflow automatisch überwacht und entsprechende Ventileinstellungen ebenfalls automatisch vorgenommen werden können.

Es ist demnach die Aufgabe der Erfindung, ein automatisch arbeitendes Insufflationsgerät zu schaffen, mit dem beispielsweise eine Hysteroskopie bei wesentlich geringerem Druck- und Gasfluß als sonst üblich durchgeführt und dennoch erreicht werden kann, daß eine ausreichende Entfaltung der Körperhöhle gewährleistet wird.

Zur Lösung dieser Aufgabe ist ein gattungsgemäßes Insufflationsgerät der eingangs erwähnten Art gemäß einem wesentlichen Aspekt der Erfindung dadurch gekennzeichnet, daß die Regelvorrichtung eine Automatikeinheit aufweist, die so eingerichtet ist, daß bei einem Automatikbetrieb des Geräts für die Fälle, daß ein Drucksensor während des Insufflierens entweder einen Druckabfall erfaßt oder erkennt, daß der erfaßte Druck nicht mehr zunimmt, die Automatikeinheit den Druck so lange vermindert, bis der gemessene Gasflow gegen Null geht.

Dabei können der Flow- und der Druckwert, bei denen der Druckabfall oder das Konstantbleiben des Drucks erfaßt wird, auch jeden Wert unterhalb der erwähnten Maximalwerte haben.

Dagegen kann mit der Vorrichtung nach DE-C-3 611 018 (vgl. Figuren 4a, b, c) vielmehr nur eine automatisierte und getaktete Druckmessung vorgenommen werden, wobei, um überhaupt den korrekten Körperhöhlendruck messen zu können, für diese Messung der Gasflow zu Null gemacht werden muß. Im übrigen kann bei den gegebenen Druck- und Flowwerten der vorbekannten Vorrichtung beim vorhandenem Flow niemals der tatsächliche intraabdominelle Druck gemessen werden.

Grundsätzlich sind die Regeleigenschaften des Insufflationsgeräts im Normalbetrieb und im Automatikbetrieb gleich. Beide Parameter, Gasdruck und -flow, beeinflussen die Ermittlung des Stellwerts über den Regelalgorithmus. Erreicht oder überschreitet einer der beiden Parameter einen jeweils vorgegebenen maximalen Grenzwert, so wird das Stellglied geschlossen. Sonst wird das Stellglied bevorzugt durch ein Proportional/Integral-Regelverhalten beeinflußt.

Lediglich im Automatikbetrieb wird durch die Automatikeinheit, nachdem der Drucksensor einen etwa durch Öffnen der in den Bauchraum führenden Tuben bedingten Druckabfall oder keinen weiteren Druckanstieg erkannt hat, der Gasflow minimiert. Also wird demnach nach der Erfassung eines Druckabfalls oder eines Gleichbleibens des Drucks dieser so lange zurückgeregelt, bis der Gasflow gegen Null geht.

Die Erfassung des Drucks durch den Sensor und die Verarbeitung in der Automatikeinheit der Regelvorrichtung machen keinen Unterschied zwischen einem Druckabfall durch Gasentweichung in den Bauchraum oder nach außen durch ein Instrument, beispielsweise ein Hysteroskop. Dies wird der untersuchende Arzt anhand anderer Kriterien feststellen müssen.

Bei einer bevorzugten Ausführungsart des erfindungsgemäßen Insufflationsgeräts sind als Sensoren mindestens ein Drucksensor und ein Flowsensor vorgesehen, welche in dieser Reihenfolge in einer Druckleitung des Insufflationsgerätes stromabwärts zwischen dem Stellglied und dem Insufflationsinstrument vorgesehen sind.

Bevorzugt ist die Signalverarbeitung der vom Drucksensor und vom Flowsensor gelieferten Signale in der Regelvorrichtung und der Automatikeinheit digital. Dabei erzeugt der Drucksensor einen dem Druck in der Druckleitung entsprechenden ersten analogen Gleichspannungswert, der durch einen in der Regelvorrichtung enthaltenen ersten Analog/Digitalwandler in einen entsprechenden ersten digitalen Istwert umgesetzt wird.

Weiterhin erzeugt der Flowsensor einen dem Gasflow in der Druckleitung entsprechenden zweiten analogen Gleichspannungswert, der durch einen zweiten Analog/Digitalwandler in der Regelvorrichtung in einen entsprechenden zweiten digitalen Istwert umgesetzt wird. Dann führt die Regelvorrichtung aufgrund des digitalen ersten und zweiten Istwerts und vorgegebener Sollwerte entsprechend einem vorgegebenen Regelalgorithmus eine digitale Verarbeitung aus, wodurch sie einen digitalen Stellwert erzeugt, der dem Stellglied zugeführt wird.

Das Stellglied läßt sich stufenlos durch ein ihm von der Regelvorrichtung zugeführtes, in der Regelvorrichtung erzeugtes impulsdauermoduliertes Signal einstellen, und das Stellglied ist bevorzugt ein solenoidgesteuertes Proportionalventil. Um ein Entweichen des Gases aus dem Cavum Uteri zu minimieren oder zu unterdrücken, wird somit aufgrund durch die Sensoren in der Druckleitung erfaßter digitaler Istwerte und vorgegebener Sollwerte von der in der Regelvorrichtung implementierten Automatikeinheit eine digitale Verarbeitung durchgeführt, durch die mittels des Stellglieds der Druck auf einen Wert zurückgeregelt wird, bei dem die Körperhöhle noch entfaltet bleibt und die Tuben nicht oder nicht mehr geöffnet sind. Durch die stufenlos einstellbare Druckregelung ist das Entweichen des insufflierten Mediums minimiert und der Patient wird schonend behandelt.

Die Regelvorrichtung weist einen Mikroprozessor auf, der die Erzeugung des jeweiligen Stellwerts aus den Sollwerten und den gemessenen Istwerten aufgrund arithmetischer Berechnungen durchführt. Alternativ dazu kann die Regelvorrichtung einen Mikroprozessor aufweisen, der mindestens einen Regelalgorithmus und gewünschte Sollwerte in Form von charakteristischen Tabellen speichert und dann aufgrund der gemessenen digital gewandelten Istwerte eine Tabellenverarbeitung zur Ermittlung des jeweiligen Stellwerts ausführt.

Am Mikroprozessor läßt sich relativ einfach eine Eingabevorrichtung vorsehen, die es gestattet, jeweilige Druck- und Flowsollwerte in vorbestimmten Grenzen variabel einzugeben. Außerdem ist mit dem Mikroprozessor eine Anzeigeeinrichtung verbindbar, die eine Sichtanzeige insbesondere der gemessenen Istwerte und auch der Sollwerte erzeugen kann. Ferner wird vorgeschlagen, den Mikroprozessor mit einer Hard- und Software-Schnittstelle zu versehen, um einen handelsüblichen Personal Computer anschließen zu können, mit dem die Funktionen des erfindungsgemäßen Insufflationsgeräts noch flexibler gestaltet werden können.

Die Erfindung wird nachstehend anhand eines Ausführungsbeispiels unter Bezug auf die beiliegende Zeichnung näher beschrieben. Es zeigen:

Fig. 1 ein Regelschema der für ein erfindungsgemäßes Insufflationsgerät vorgeschlagenen Regelvorrichtung,

Fig. 2 ein Funktions-Zeitdiagramm zur Erläuterung der wesentlichen Funktionen des Insufflationsgerätes und

Fig. 3 schematisch ein Blockschaltbild einer bevorzugten Ausführungsart eines Insufflationsgeräts.

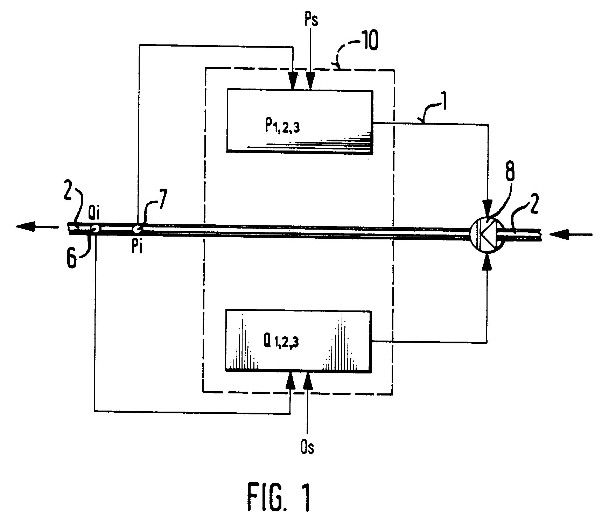

Fig. 1 stellt schematisch einen Abschnitt einer von einem Druckreservoir zu einer Patientin führenden Druckleitung 2 dar, mit der eine Regelvorrichtung 1 verbunden ist. Als Meßteile dieser Regelvorrichtung 1 sind in der Druckleitung 2 stromabwärts von einem Stellglied 8 ein Drucksensor 7 und ein Gasflowsensor 6 angeordnet, die jeweils einen Istdruck $P_i$ und einen Flowwert $Q_i$ als Istwert erfassen. Diese Istwerte $P_i$ und $Q_i$ werden einer Automatikvorrichtung 10 der Regelvorrichtung 1 zugeführt und dort durch einen Integral/Proportional-Regelalgorithmus entsprechend vorgebbarer Druck- und Flowsollwerte $P_s$, $Q_s$ verarbeitet. In der durch eine gestrichelte Linie angedeuteten Automatikeinheit 10 sind separate Regelkreise jeweils zur Erzeugung von aufeinanderfolgenden Stellwerten $p_{1,2,3}$ und $Q_{1,2,3}$ enthalten, die dem Stellglied 8 zugeführt werden. Die gesamte Regelvorrichtung führt eine sogenannte Rückwärtsregelung aus, da das Stellglied 8 in der Druckleitung 2 stromaufwärts von den Sensoren 6 und 7 liegt.

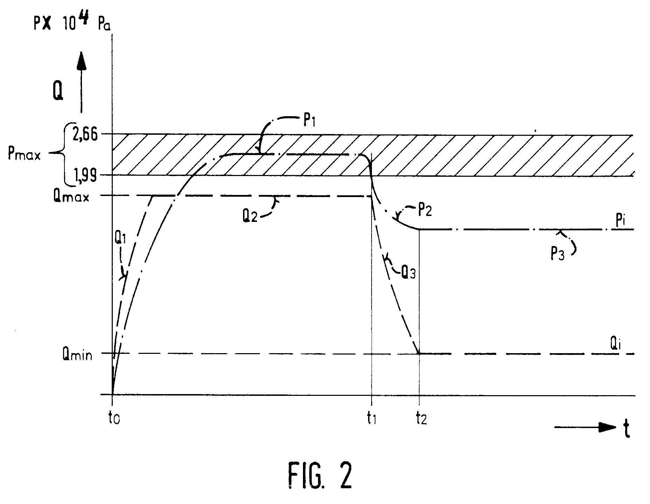

Anhand des Diagramms in Fig. 2 werden nun die erfindungswesentlichen Funktionen des Insufflationsgeräts erläutert. In Fig. 2 ist die Zeit t auf der Abszisse und der Druck P und der Gasflow Q auf der Ordinate aufgetragen. Zwischen den Druckwerten 1,99 und 2,66 x 10$^4$ Pa ist schraffiert ein Maximalbereich angedeutet, den der Druck im Hohlraum des Uterus nicht überschreiten darf. Der idealisierte Verlauf des Drucks P$_i$, wie ihn der Drucksensor 7 erfaßt, ist durch eine strichpunktierte Kurve wiedergegeben, die in drei Abschnitte P$_1$, P$_2$ und P$_3$ unterteilt ist. Eine gestrichelte Kurve Q$_i$ die die Abschnitte Q$_1$, Q$_2$ und Q$_3$ aufweist, deutet den Verlauf des Gasflows an.

Ausgehend von einem Zeitpunkt t$_0$, zu dem der Insufflationsvorgang gestartet werden soll, steigen der Gasflow Q$_i$ und der Verlauf des Drucks P$_i$ in der Druckleitung 2 zunächst steil an. Der Gasflow und der Druck erreichen nach kurzer Zeit jeweils einen Maximalwert, der für den Druck in dem schraffierten Bereich zwischen 1,99 und 2,66 x 10$^4$ Pa liegt. Der Maximalwert des Gasflows Q$_{max}$ darf dagegen 100 ml/min nicht überschreiten. Die Regelvorrichtung sorgt also zunächst dafür, daß der in der Körperhöhle gemessene Druck P$_i$ und der Gasflow Q$_i$ die jeweiligen maximalen Werte P$_{max}$ und Q$_{max}$ nicht überschreiten (Zweige P$_1$ und Q$_2$). Tritt zu irgendeinem Zeitpunkt, beispielsweise bei t$_1$, ein Druckabfall auf, der z.B. durch das Öffnen der Tuben im Körper einer Patientin verursacht sein kann, so sorgt im Automatikbetrieb die Automatikeinheit 10 dafür, daß der Druck, ausgehend von dem höchsten Druckwert P$_1$, vermindert wird (Zweig P$_2$), bis der gemessene Gasflow gegen Null geht (Zweig Q$_3$) oder einen Minimalwert Q$_{min}$ unterschreitet. Dies ist in Fig. 2 z. B. zu einem Zeitpunkt t$_2$ geschehen. Ab diesem Zeitpunkt t$_2$ wird dann der Druck wieder konstant gehalten (Zweig P$_3$), um in diesem Zustand die gewünschten Untersuchungen oder Eingriffe in dem Körperhohlraum durchführen zu können.

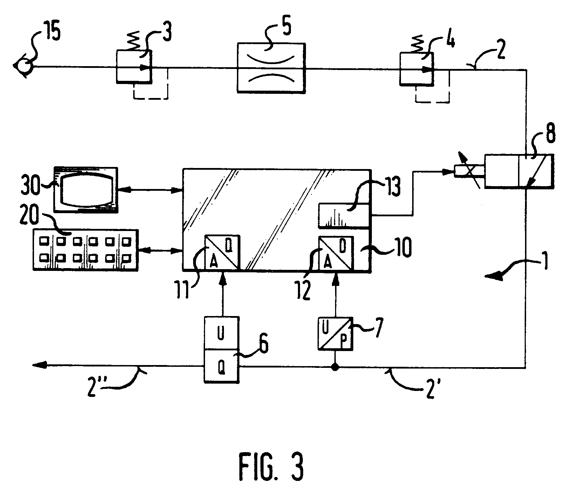

Gemäß Fig. 3 führt eine Gasdruckleitung, die in Abschnitte 2, 2' und 2" unterteilt ist, von einem Gasreservoir 15 über einen ersten Druckminderer 3, eine Flowdrossel 5, einen zweiten Druckminderer 4, ein Proportionalventil 8, einen Drucksensor 7 und einen Flowsensor 6 z.B. über einem Hysteroskopadapter zur Patientin. Der dem Gasreservoir 15 nachgeschaltete erste Druckminderer 3 setzt den Druck herab. Die Flowdrossel 5 ist zur Begrenzung des maximalen Durchflusses in die Gasdruckleitung 2 eingeschaltet. Der zweite Druckminderer 4 begrenzt auf einen maximal zulässigen Druck. Das Proportionalventil 8 wird von der den Mikroprozessor 10 aufweisenden Regelvorrichtung gesteuert, und zwar je nach den von dem Drucksensor 7 und dem Flowsensor 6 erfaßten Druck- und Gasdurchfluß-Istwerten und vorgegebenen Sollwerten.

Zum Mikroprozessor 10 gehört ein erster Analog/Digitalwandler 11, der die vom Flowsensor 6 erzeugte, dem Istflowwert entsprechende Gleichspannung U$_Q$ in ein entsprechendes Digitalsignal umsetzt, ein zweiter Analog/Digitalwandler 12, der die vom Drucksensor 7 erzeugte, dem Istdruck entsprechende Gleichspannung U$_p$ in einen entsprechenden Digitalwert umsetzt, und ein Modulator 13, der ein impulsdauermoduliertes Stellsignal erzeugt, welches dem als Magnetventil ausgebildeten Proportionalventil 8 zugeführt wird. Ferner sind mit dem Mikroprozessor 10 eine Eingabevorrichtung 20 und eine Displayvorrichtung 30 verbunden. Mittels der Eingabevorrichtung 20 können Systemparameter, wie Druck- und Flowsollwerte, gewünschte Regelalgorithmen, Zeitgrößen und Steuersignale zum Starten und Beenden des Betriebs des Insufflationsgeräts eingegeben werden. Die Displayvorrichtung 30 erzeugt eine Sichtanzeige von Daten, insbesondere von Soll- und Istwerten, der Regelvorrichtung 1.

Nachfolgend wird im einzelnen die grundsätzliche Signalverarbeitung der Sensorsignale beschrieben.

Drucksensor

Der im Abschnitt 2' der Druckleitung erfaßte Istdruck P$_i$ wird durch den Drucksensor 7 in eine dazu proportionale Spannung

$$U_p = P_i \times K_p,$$

umgewandelt, worin K$_p$ ein Proportionalitätsfaktor ist, der die Einheit Volt/Pa oder Volt/mmHg oder Volt/mbar je nach verwendetem Drucksensor hat.

Aus der Spannung U$_p$ wird der digitale Istwert in dem zweiten Analog/Digitalwandler 12 gebildet und im Mikroprozessor der Regelvorrichtung verarbeitet.

Daraus wird die digitale Stellgröße X$_p$ nach folgender Gleichung ermittelt:

$$X_p = [P_s - (P_i - \text{Offset})] \times \text{Regelkonstante}.$$

Darin stellen P$_s$ den digitalen Sollwert des Druckes, P$_i$ den digitalen Istwert des Druckes, Offset einen vom Drucksensor abhängigen Wert und die Regelkonstante einen vom Prozessorsystem, der Programmiersprache und dem Stellglied abhängigen konstanten Wert dar.

Flowsensor

Der Flowsensor 6 erzeugt die dem Flowert Q$_i$ entsprechende Spannung U$_Q$ nach folgender Beziehung:

$$U_Q = Q_i \times K_q.$$

K$_q$ ergibt sich aus der nichtlinearen Kennlinie des verwendeten Flowsensors und ist deshalb selbst abhängig von Q.

Aus der nach der obigen Beziehung erhaltenen Flowspannung U$_Q$ wird vom ersten Analog/Digitalwandler 11 der digitale Istwert gebildet und von der Regelvorrichtung verarbeitet. Daraus wird dann eine digitale Stellgröße X$_Q$ nach folgender Beziehung ermittelt:

$$X_Q = [Q_s - (Q_i - \text{Offset})] \times \text{Regelkonstante},$$

worin $Q_s$ den digitalen Sollwert und $Q_i$ den digitalen Istwert des Flows, Offset einen vom Flowsensor abhängigen Wert und die Regelkonstante eine vom Prozessorsystem, der Programmiersprache und dem Stellglied abhängige Regelkonstante angeben.

Abhängig davon, welche der Stellgrößen dominiert, wird $X_p$ oder $X_q$ in ein entsprechendes impulsdauermoduliertes Signal durch den Modulator 13 im Mikroprozessor 10 umgesetzt und als solches dem Proportionalventil 8 zugeführt. Das Regelverfahren zur Einstellung des Ventils 8 weist bevorzugt ein Proportional/Integral-Verhalten auf.

Die zum Mikroprozessor 10 gehörende Automatikeinheit hat im Automatikbetrieb, wenn dieser z.B. durch die Eingabevorrichtung 20 initiiert wurde, die bereits anhand der Fig. 2 beschriebene Funktion. Dabei wird jeder Druckabfall erkannt, egal ob dieser durch das Entweichen des Gases in den Bauchraum oder durch ein Instrument verursacht wurde. Im Normalbetrieb, d.h. nicht im Automatikbetrieb, wird das Stellglied, wenn einer der beiden Istwerte Druck oder Flow seinen maximal vorgegebenen Grenzwert überschreitet, geschlossen. Bei sonstigen Werten der beiden Istwerte Druck und Flow wird das Stellglied 8 durch ein Proportional/Integral-Verhalten beeinflußt. Dadurch, daß im Automatikbetrieb durch die Automatikeinheit im Mikroprozessor 10 während der Insufflation auf einen niedrigeren Druck geregelt wird, wenn irgendein Druckabfall nach dem Entfalten der Körperhöhle erfaßt wird, ist sichergestellt, daß die Patientin nicht unnötig belastet wird und dennoch die Körperhöhle entfaltet bleibt.

## Patentansprüche

1. Insufflationsgerät, insbesondere zum Insufflieren von Gas in das Cavum Uteri, mit einem Gas liefernden Reservoir (15) und mit einer Regelvorrichtung (1), mit der Sensoren (6, 7) und ein Stellglied (8) verbunden sind, um den Druck und den Flow des in eine Körperhöhle eingeleiteten Gases zu messen, auf vorgebbare Werte einzustellen und um die Gaszufuhr nach Erreichen eines als Maximalwert vorgebbaren Gasdrucks ($P_{max}$) in der Körperhöhle zu beenden, **dadurch gekennzeichnet**, daß die Regelvorrichtung (1) eine Automatikeinheit (10) aufweist, die so eingerichtet ist, daß bei einem Automatikbetrieb des Geräts für Fälle, daß ein Drucksensor (7) während des Insufflierens entweder einen Druckabfall erfaßt oder erkennt, daß der erfaßte Druck nicht mehr zunimmt, der Druck so lange vermindert wird, bis der gemessene Gasflow (Q) gegen Null geht.

2. Insufflationsgerät nach Anspruch 1, dadurch gekennzeichnet, daß als Sensoren (6, 7) mindestens ein Drucksensor (7) und ein Flowsensor (6) vorgesehen sind, die in dieser Reihenfolge in einer Druckleitung (2', 2'') des Insufflationsgeräts stromabwärts zwischen dem Stellglied (8) und einem Insufflationsinstrument vorgesehen sind.

3. Insufflationsgerät nach Anspruch 1 oder 2, dadurch gekennzeichnet, daß der Drucksensor (7) einen dem Druck in der Druckleitung (2, 2') entsprechenden ersten analogen Gleichspannungswert ($U_p$) erzeugt und daß die Regelvorrichtung (1) digital arbeitet und einen ersten Analog/Digitalwandler (12) aufweist, der den ersten analogen Gleichspannungswert ($U_p$) in einen entsprechenden ersten digitalen Istwert umsetzt.

4. Insufflationsgerät nach einem der Ansprüche 1 bis 3, dadurch gekennzeichnet, daß der Flowsensor (6) einen dem Gasflow (Q) in der Druckleitung (2', 2'') entsprechenden, zweiten analogen Gleichspannungswert ($U_Q$) erzeugt und die digitale Regelvorrichtung (1) einen zweiten Analog/Digitalwandler (11) aufweist, der den zweiten analogen Gleichspannungswert ($U_Q$) in einen entsprechenden zweiten digitalen Istwert umsetzt.

5. Insufflationsgerät nach einem der Ansprüche 1 bis 4, dadurch gekennzeichnet, daß die digital arbeitende Regelvorrichtung (1) den erwähnten ersten und den zweiten digitalen Istwert entsprechend einem vorgegebenen Regelalgorithmus und vorgegebener Sollwerte verarbeitet und einen digitalen Stellwert erzeugt, der dem Stellglied (8) zugeführt wird.

6. Insufflationsgerät nach einem der Ansprüche 1 bis 5, dadurch gekennzeichnet, daß die Regelvorrichtung (1) eine Impulsdauermodulationseinrichtung (13) aufweist, die den digitalen Stellwert in Form eines impulsdauermodulierten Signals erzeugt, und daß das Stellglied (8) ein solenoidgesteuertes Ventil ist.

7. Insufflationsgerät nach einem der Ansprüche 1 bis 6, dadurch gekennzeichnet, daß die Regelvorrichtung (1) einen Mikroprozessor (10) aufweist, der den jeweiligen Stellwert aus den Sollwerten und den von den Sensoren gemessenen Istwerten aufgrund arithmetischer Berechnungen ermittelt.

8. Insufflationsgerät nach einem der Ansprüche 1 bis 6, dadurch gekennzeichnet, daß die Regelvorrichtung (1) einen Mikroprozessor (10) aufweist, in dem mindestens ein Regelalgorithmus und Sollwerte in Tabellenform gespeichert sind, und daß der Mikroprozessor eine Tabellenverarbeitung zur Ermittlung des jeweiligen Stellwerts ausführt.

9. Insufflationsgerät nach einem der Ansprüche 1 bis 8, dadurch gekennzeichnet, daß eine mit dem Mikroprozessor (10) verbundene Eingabevorrichtung (20) vorgesehen ist, durch die jeweilige Druck- und Flowsollwerte in vorbestimmten Grenzen variabel eingebbar sind.

10. Insufflationsgerät nach einem der Ansprüche 1 bis 9, dadurch gekennzeichnet, daß eine mit dem Mikroprozessor (10) verbundene Anzeigeeinrichtung (30) vorgesehen ist, die eine Sichtanzeige der jeweils von den Sensoren gemessenen Istwerte und der Sollwerte erzeugt.

11. Insufflationsgerät nach einem oder mehreren der Ansprüche 1 bis 10, dadurch gekennzeichnet, daß die Automatikeinheit im Mikroprozessor (10) implementiert ist.

12. Insufflationsgerät nach einem oder mehreren der Ansprüche 1 bis 11, dadurch gekennzeichnet, daß der Mikroprozessor (10) eine Hard- und Software-Schnittstelle zum Anschluß an einen handelsüblichen Personal Computer aufweist.

**Claims**

1. An insufflation apparatus, in particular for insufflating gas into the Cavum Uteri, with a reservoir (15) delivering a gas and with a control device (1) to which sensors (6, 7) and an actuator (8) are connected in order to measure the pressure and the flow of the gas introduced into the body cavity, to set the pressure and flow to presettable values and in order to end the supply of gas after reaching a gas pressure ($P_{max}$) in the body cavity, which can be set as the maximum value, **characterised in that** the control device (1) comprises an automatic unit (10) which is set up such that with an automatic operation of the apparatus for cases that a pressure sensor (7) during the insufflation either acquires or recognises a fall in pressure, that the acquired pressure no longer increases, the pressure is reduced for so long until the measured gasflow (Q) approaches zero.

2. An insufflation apparatus according to claim 1, characterised in that as sensors (6, 7) at least one pressure sensor (7) and a flow sensor (6) are provided which in this sequence are provided in a pressure conduit (2', 2") of the insufflation apparatus downstream between the actuator (8) and an insufflation instrument.

3. An insufflation apparatus according to claim 1 or 2, characterised in that the pressure sensor (7) produces a first analog constant voltage value ($U_p$) corresponding to the pressure in the pressure conduit (2', 2") and that the control device (1) functions digitally and has a first analog/digital coverter (12) which converts the first analog constant voltage value ($U_p$) into a corresponding first digital actual value.

4. An insufflation apparatus according to one of the claims 1 to 3, characterised in that the flow sensor (6) produces a second analog constant voltage value ($U_Q$) corresponding to the gas flow (Q) in the pressure conduit (2, 2") and the digital control device (1) comprises a second analog/digital converter (11) which converts the second analog constant voltage value ($U_Q$) into a corresponding second digital actual value.

5. An insufflation apparatus according to one of the claims 1 to 4, characterised in that the digitally functioning control device (1) processes the mentioned first and second digital actual value corresponding to a preset control algorithm and a preset nominal value and produces a digital correcting value which is supplied to the actuator (8).

6. An insufflation apparatus according to one of the claims 1 to 5, characterised in that the control device (1) comprises an impulse duration modulated signal and that the actuator (8) is a valve controlled by solenoid.

7. An insufflation apparatus according to one of the claims 1 to 6, characterised in that the control device (1) comprises a microprocessor (10) which determines the respective correcting value from the nominal values and the actual values measured by the sensors, on account of arithmetical computations.

8. An insufflation apparatus according to one of the claims 1 to 6, characterised in that the control device (1) comprises a microprocessor (10) in which at least one control algorithm and nominal values in table form are stored, and that the microprocessor carries out a tabular processing for determining the respective correcting value.

9. An insufflation apparatus according to one of the claims 1 to 8, characterised in that an input device (20) connected to the microprocessor (10) is provided via which the respective pressure and flow nominal values can be variably inputted in predetermined limits.

10. An insufflation apparatus according to one of the claims 1 to 9, characterised in that there is provided a display means (30) connected to the microprocessor, which produces a visible display of the actual values measured by the sensors in each case and of the nominal values.

11. An insufflation apparatus according to one or more of the claims 1 to 10, characterised in that the automatic unit is implemented into the microprocessor (10).

12. An insufflation apparatus according to one or more of claims 1 to 11, characterised in that the microprocessor (10) comprises a hardware and software interface for connection to a commercially available personal computer.

**Revendications**

1. Appareil d'insufflation, en particulier pour insuffler du gaz dans la cavité utérine, comprenant un réservoir (15) délivrant du gaz et un mécanisme de réglage (1) auquel sont reliés des capteurs (6, 7) et un élément fonctionnel du dispositif de réglage (8) pour mesurer la pression et l'écoulement du gaz introduit dans une cavité corporelle et pour les régler à des valeurs prédéfinissables et pour mettre un terme à l'alimentation en gaz après avoir atteint une pression de gaz ($P_{max}$) prédéfinissable à titre de valeur maximale dans la cavité corporelle, caractérisé en ce que le dispositif de réglage (1) présente une unité de commande automatique (10) montée de telle sorte que, lors d'un fonctionnement automatique de l'appareil, dans des cas où un capteur de pression (7) au cours de l'insufflation, soit enregistre une chute de pression, soit constate que la pression enregistrée n'augmente plus, elle diminue la pression jusqu'à ce que l'écoulement de gaz mesuré (Q) atteigne la valeur zéro.

2. Appareil d'insufflation selon la revendication 1, caractérisé en ce qu'on prévoit, à titre de capteurs (6, 7), au moins un capteur de pression (7) et un capteur de l'écoulement (6) qui sont prévus dans cet ordre dans un conduit de pression (2', 2'') de l'appareil d'insufflation, en aval entre l'élément fonctionnel du dispositif de réglage (8) et un instrument d'insufflation.

3. Appareil d'insufflation selon la revendication 1 ou 2, caractérisé en ce que le capteur de pression (7) génère une première valeur de tension continue analogique ($U_p$) correspondant à la pression régnant dans le conduit de pression (2, 2') et en ce que le dispositif de réglage (1) travaille de manière numérique et présente un convertisseur analogique/numérique (12) qui convertit la première valeur de tension continue analogique ($U_p$) en une première valeur réelle numérique correspondante.

4. Appareil d'insufflation selon l'une quelconque des revendications 1 à 3, caractérisé en ce que le capteur de l'écoulement (6) génère une seconde valeur de tension continue analogique ($U_q$) correspondant à l'écoulement de gaz (Q) dans le conduit de pression (2', 2'') et le dispositif de réglage numérique (1) présente un second convertisseur analogique/numérique (11) qui convertit la seconde valeur de tension continue analogique ($U_q$) en une seconde valeur réelle numérique correspondante.

5. Appareil d'insufflation selon l'une quelconque des revendications 1 à 4, caractérisé en ce que le dispositif de réglage (1) travaillant de manière numérique traite la première et la seconde valeur réelle numérique mentionnée en fonction d'un algorithme de réglage prédéfini et en fonction de valeurs de consigne prédéfinies et génère une valeur de réglage numérique qui est acheminée à l'élément fonctionnel du dispositif de réglage (8).

6. Appareil d'insufflation selon l'une quelconque des revendications 1 à 5, caractérisé en ce que le dispositif de réglage (1) présente un mécanisme de modulation (13) de la durée d'impulsion qui génère la valeur de réglage numérique sous la forme d'un signal à modulation par impulsions et en ce que l'élément fonctionnel du dispositif de réglage (8) est une soupape commandée par un solénoïde.

7. Appareil d'insufflation selon l'une quelconque des revendications 1 à 6, caractérisé en ce que le dispositif de réglage (1) présente un microprocesseur (10) qui détermine la valeur de réglage respective à partir des valeurs de consigne et à partir des valeurs réelles mesurées par les capteurs, sur base de calculs arithmétiques.

8. Appareil d'insufflation selon l'une quelconque des revendications 1 à 6, caractérisé en ce que le dispositif de réglage (1) présente un microprocesseur (10) dans lequel sont mémorisés au moins un algorithme de réglage et des valeurs de consigne sous forme de tables et en ce que le microprocesseur met en oeuvre une consultation des tables pour déterminer la valeur de réglage respective.

9. Appareil d'insufflation selon l'une quelconque des revendications 1 à 8, caractérisé en ce qu'on prévoit un dispositif d'entrée (20) lié au microprocesseur (10) par lequel on peut entrer de manière variable dans des limites prédéfinies les valeurs de consigne de pression et d'écoulement respectives.

10. Appareil d'insufflation selon l'une quelconque des revendications 1 à 9, caractérisé en ce qu'on prévoit un mécanisme d'affichage (30) relié au microprocesseur (10), qui génère un affichage visuel des valeurs réelles respectivement mesurées par les capteurs et des valeurs de consigne.

11. Appareil d'insufflation selon une ou plusieurs des revendications 1 à 10, caractérisé en ce que l'unité de commande automatique est incorporée dans le microprocesseur (10).

12. Appareil d'insufflation selon une ou plusieurs des revendications 1 à 11, caractérisé en ce que le microprocesseur (10) présente une interface de matériel et de logiciel à des fins de raccordement à un ordinateur personnel disponible dans le commerce.

FIG. 1

FIG. 2

FIG. 3